(19) Europäisches Patentamt
European Patent Office
Office européen des brevets

(11) Publication number: **0 094 181 A1**

# (12) EUROPEAN PATENT APPLICATION

(21) Application number: 83302430.0

(22) Date of filing: 29.04.83

(51) Int. Cl.³: **A 01 N 43/82**
// C07D417/04, C07D285/12

(30) Priority: 03.05.82 US 374137

(43) Date of publication of application: 16.11.83
Bulletin 83/46

(84) Designated Contracting States: BE CH DE FR GB IT LI LU NL SE

(71) Applicant: ELI LILLY AND COMPANY, 307, East McCarty Street, Indianapolis Indiana 46285 (US)

(72) Inventor: Burow, Kenneth Wayne, Jr., 6467 Johnson Road, Indianapolis Indiana 46220 (US)
Inventor: Thibault, Thomas Delor, 1520 N. Franklin Road, Indianapolis Indiana 46219 (US)

(74) Representative: Crowther, Terence Roger et al, Erl Wood Manor, Windlesham Surrey GU20 6PH (GB)

(54) **Use of thiadiazolylimidazolidinones to control aquatic vegetation.**

(57) Thiadiazolylimidazolidinones are useful in the control of aquatic vegetation.

EP 0 094 181 A1

# Use of Thiadiazolylimidazolidinones to Control Aquatic Vegetation

This invention concerns methods for controlling the growth of vegetation in bodies of water.

A large number of thiadiazolylimidazolidinone derivatives are known in the art as terrestrial herbicides. Krenzer, for example, in U.S. Patent No. 3,964,895, described the very potent terrestrial herbicide buthidazole.

While there are numerous chemical agents currently available that are effective as selective terrestrial herbicides, few agents are available for controlling the growth of aquatic vegetation in water bodies such as lakes, ponds, streams, rivers and the like. Most herbicides that are effective terrestrially are not suitable for use in aquatic environments. This may be due to the fact that the terrestrial herbicides simply will not control the aquatic vegetation, the terrestrial herbicide is not stable in an aquatic environment, or because the toxicity of the terrestrial herbicides render them unfit for use in water containing animal life.

To date, none of the known thiadiazolylimidazolidinones have been used to control the growth of aquatic plants. We have discovered that certain thiadiazolylimidazolidinones can be used to control the growth of aquatic vegetation.

Specifically, this invention is directed to a method for controlling the growth of aquatic plants comprising contacting the plants or the water in which the plants are growing with an aquatic herbicidally-effective amount of a thiadiazolylimidazolidinone derivative defined by the formula (I):

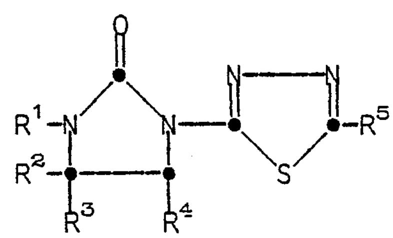

(I)

wherein:

$R^1$ is hydrogen, $C_1$-$C_6$ alkyl, $C_2$-$C_6$ alkenyl, $C_2$-$C_6$ alkynyl, or halo-$C_1$-$C_6$ alkyl;

$R^2$ is hydrogen, or taken together with $R^4$ completes a double bond.

$R^3$ is hydrogen, $C_1$-$C_6$ alkyl, hydroxy, $C_1$-$C_6$ alkoxy or $C_1$-$C_6$ alkanoyloxy;

$R^4$ is hydroxy, $C_1$-$C_6$ alkoxy, $C_1$-$C_6$ alkanoyloxy, or taken together with $R^2$, $R^4$ completes a double bond;

$R^5$ is $C_1$-$C_6$ alkyl, $C_2$-$C_6$ alkenyl, $C_2$-$C_6$ alkynyl, halo-$C_1$-$C_6$ alkyl, $C_1$-$C_6$ alkylthio, $C_1$-$C_6$ alkylsulfinyl, $C_1$-$C_6$ alkylsulfonyl, or $SO_2NR^6R^7$, wherein $R^6$ is hydrogen or $C_1$-$C_6$ alkyl and $R^7$ is $C_1$-$C_6$ alkyl, $C_1$-$C_6$ alkoxy, $C_2$-$C_6$ alkenyl, or $C_2$-$C_6$ alkynyl.

A preferred method for controlling aquatic vegetation employs a compound of formula (I) wherein $R^1$ is $C_1$-$C_6$ alkyl, $R^2$ and $R^3$ both are hydrogen, $R^4$ is hydroxy and $R^5$ is $C_1$-$C_6$ alkyl or $SO_2NR^6R^7$ where $R^6$ and $R^7$ independently are $C_1$-$C_6$ alkyl.

Another preferred embodiment employs a compound of formula (I) wherein $R^5$ is 1,1-dimethylethyl.

A further embodiment of this invention is an aquatic herbicide formulation comprising a thiadiazolylimidazolidinone of the formula (I) as an active ingredient, associated with an aquatically-acceptable carrier.

$R^1$ in formula (I) includes a $C_1$-$C_6$ alkyl group. The term "$C_1$-$C_6$ alkyl" means both straight and branched carbon chains having up to six carbon atoms. Typical $C_1$-$C_6$ alkyl groups include methyl, ethyl, isopropyl, n-butyl, 2-methylpentyl, 1,2-dimethylpropyl, and the like. Methyl is preferred for $R^1$.

The term "halo" as used herein means fluoro, chloro, bromo and iodo.

Typical $C_2$-$C_6$ alkenyl groups include 2-propenyl, 2-butenyl, 2-methyl-3-pentenyl, and 5-hexenyl. Common $C_2$-$C_6$ alkynyl groups include 3-hexynyl, 2-propynyl, 2-butynyl, 1-methyl-2-butynyl and the like.

The term "halo-$C_1$-$C_6$ alkyl" means a $C_1$-$C_6$ alkyl group substituted by one or more halo groups. Trifluoromethyl is a preferred haloalkyl group. Other haloalkyl groups are 2-chloroethyl, 3,4-dibromopentyl, 2-bromo-3-iodohexyl, 1,1-dimethyl-2-chloroethyl, and the like.

The term "$C_1$-$C_6$ alkoxy" means a $C_1$-$C_6$ alkyl group linked through an oxygen atom. Typical $C_1$-$C_6$ alkoxy groups include methoxy, ethoxy, isopropoxy, n-butoxy, n-hexyloxy, and the like. Similarly, "$C_1$-$C_6$ alkanoyloxy" means a $C_1$-$C_6$ alkanoic acid acyl residue linked through an oxygen atom. Such $C_1$-$C_6$ alkanoyloxy groups include formyloxy, acetoxy, isobutyroxy, n-hexanoyloxy and the like.

$R^5$ in the above formula includes $C_1$-$C_6$ alkylthio, $C_1$-$C_6$ alkylsulfinyl, and $C_1$-$C_6$ alkylsulfonyl. These terms mean, respectively, a $C_1$-$C_6$ alkyl group linked through a sulfur atom, a sulfinyl group $(\overset{O}{\overset{\parallel}{S}})$, and a sulfonyl group 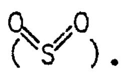. Typical of such groups are methylthio, n-pentylthio, ethylsulfinyl, isopropylsulfinyl, n-butylsulfonyl, tert.-butylsulfonyl, n-hexylsulfonyl, and the like.

The term "$SO_2NR^6R^7$" defines an aminosulfonyl substituent such as methylaminosulfonyl, diethylaminosulfonyl, N-methyl-N-hexylaminosulfonyl, N-methyl-N-(2-propenyl)aminosulfonyl, N-ethyl-N-(3-hexynyl)aminosulfonyl, and the like.

Most of the thiadiazolylimidazolidinones employed in the aquatic method of this invention are known in the herbicide art. For example, compounds defined by the formula (II)

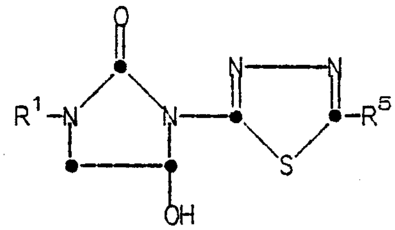

(II)

wherein $R^1$ is alkyl and $R^5$ is alkyl, alkenyl, haloalkyl, alkylthio, alkylsulfonyl or alkylsulfinyl are described in detail in U.S. Patent Nos. 3,901,904, 3,904,640, 3,964,895, 4,093,443 and 4,028,375. These patents are incorporated herein by reference for their teaching of the synthesis of such compounds.

Similarly, compounds of the general formula (III)

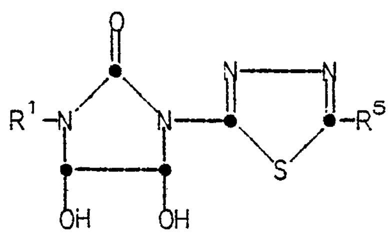

(III)

wherein $R^1$ is alkyl, alkenyl or alkynyl and $R^5$ is alkyl, haloalkyl, alkenyl, alkynyl, alkylthio, alkylsulfinyl or alkylsulfonyl are described in detail in U.S. Patent Nos. 3,758,492, 3,759,939, and 3,849,432. These patents are incorporated herein by reference for their teaching of the synthesis of such compounds.

Thiadiazolylimidazolidinone derivatives having the above general formula wherein $R^5$ is $SO_2NR^6R^7$

are novel compounds and their synthesis is described in detail below.

Most of the compounds to be employed in the aquatic method of this invention are readily prepared by general methods known in the art. The typical synthesis of a thiadiazolylimidazolidinone herbicide follows the following reaction scheme:

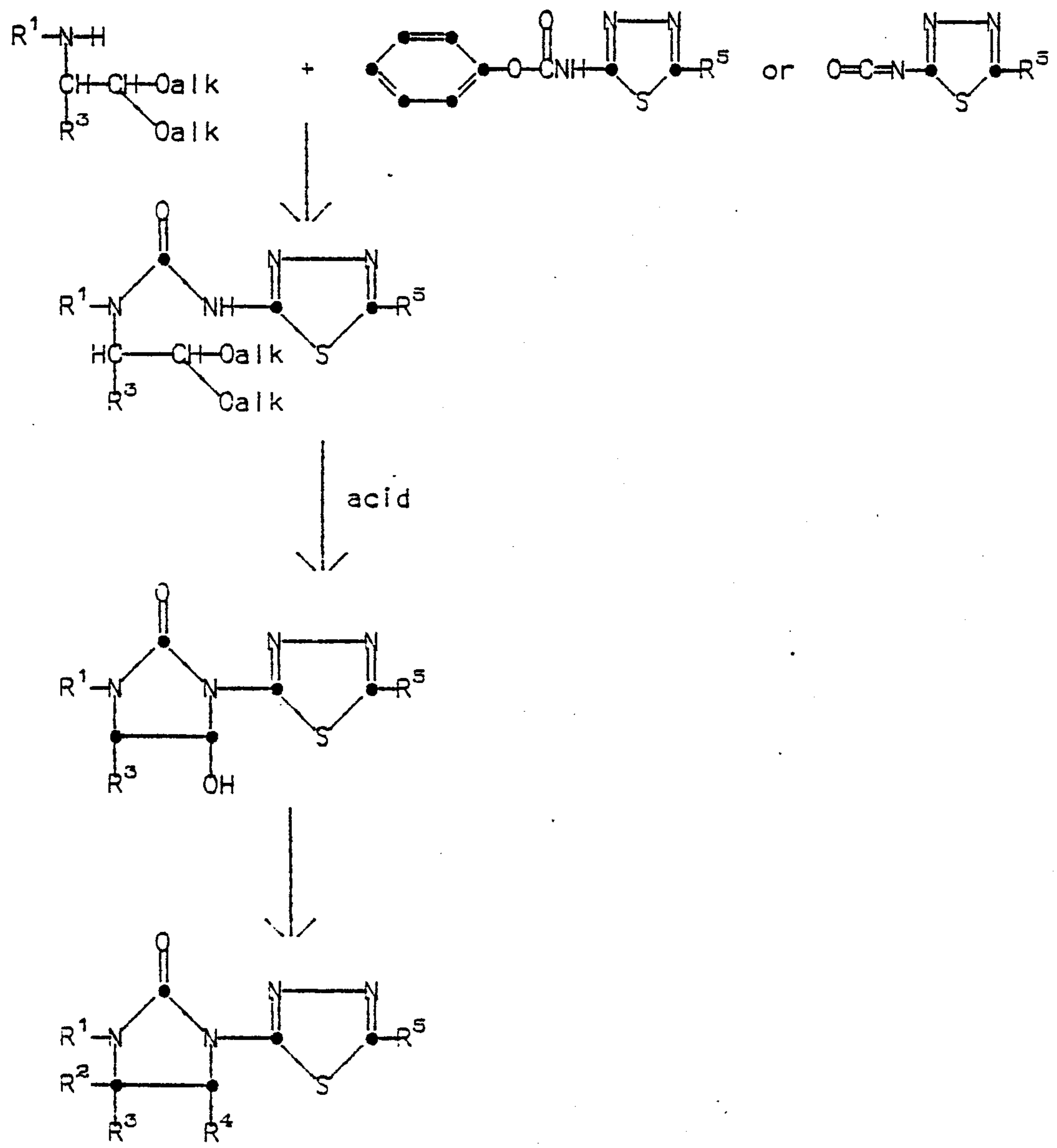


wherein $R^1$, $R^2$, $R^3$, $R^4$ and $R^5$ are as defined above, and "alk" means lower alkyl such as methyl or ethyl.

Compounds of the above formula wherein $R^3$ is hydroxy, alkanoyloxy or alkoxy are preferably prepared according to the following sequence:

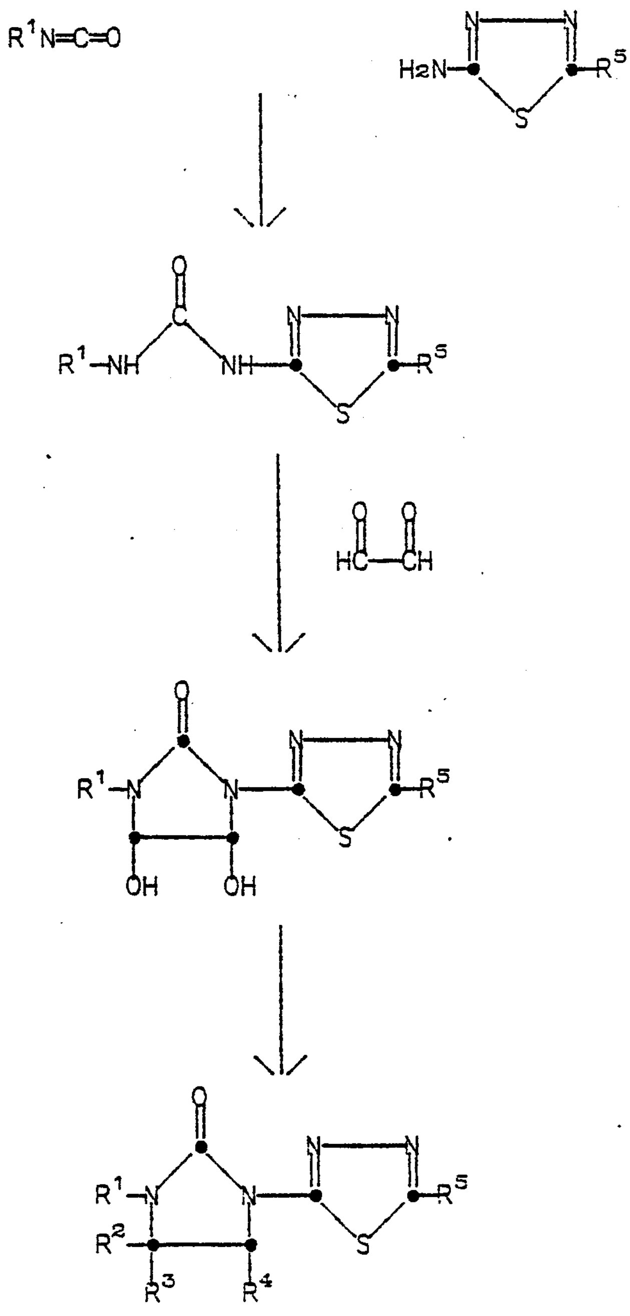

Examples of typical classes of thiadiazolylimidazolidinones to be employed in the aquatic method of this invention include those listed below:

A. Those of the formula (IV):

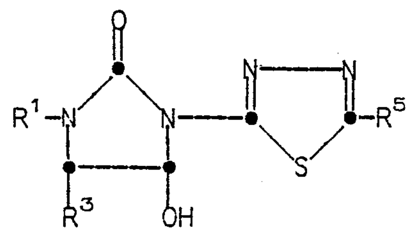
(IV)

1. $R^1$ is $C_1$-$C_6$ alkyl.

a. $R^3$ is hydrogen.

1a1. $R^5$ is $C_1$-$C_6$ alkyl.

1a2. $R^5$ is tert.-butyl.

1a3. The compound 3-(5-tert.-butyl-1,3,4-thiadiazol-2-yl)-4-hydroxy-1-methyl-2-imidazolidinone.

1a4. $R^5$ is halo-$C_1$-$C_6$ alkyl.

1a5. $R^5$ is $C_2$-$C_6$ alkenyl.

1a6. $R^5$ is $C_2$-$C_6$ alkynyl.

1a7. $R^5$ is $C_1$-$C_6$ alkylthio.

1a8. $R^5$ is $C_1$-$C_6$ alkylsulfinyl.

1a9. $R^5$ is $C_1$-$C_6$ alkylsulfonyl.

1a10. $R^5$ is $SO_2NR^6R^7$.

1a11. $R^5$ is $SO_2NR^6R^7$ where $R^6$ and $R^7$ independently are $C_1$-$C_6$ alkyl.

b. $R^3$ is $C_1$-$C_6$ alkyl.

1b1. $R^5$ is $C_1$-$C_6$ alkyl.

1b2. $R^5$ is halo-$C_1$-$C_6$ alkyl.

1b3. $R^5$ is $C_2$-$C_6$ alkenyl.

1b4. $R^5$ is $C_2$-$C_6$ alkynyl.

1b5. $R^5$ is $C_1$-$C_6$ alkylthio.

1b6. $R^5$ is $C_1$-$C_6$ alkylsulfinyl.

1b7. $R^5$ is $C_1$-$C_6$ alkylsulfonyl.
1b8. $R^5$ is $SO_2NR^6R^7$.
c. $R^3$ is hydroxy.
1c1. $R^5$ is $C_1$-$C_6$ alkyl.
1c2. $R^5$ is halo-$C_1$-$C_6$ alkyl.
1c3. $R^5$ is $C_2$-$C_6$ alkenyl.
1c4. $R^5$ is $C_2$-$C_6$ alkynyl.
1c5. $R^5$ is $C_1$-$C_6$ alkylthio.
1c6. $R^5$ is $C_1$-$C_6$ alkylsulfinyl.
1c7. $R^5$ is $C_1$-$C_6$ alkylsulfonyl.
1c8. $R^5$ is $SO_2NR^6R^7$.

2. $R^1$ is $C_2$-$C_6$ alkenyl.
a. $R^3$ is hydrogen.
2a1. $R^5$ is $C_1$-$C_6$ alkyl.
2a2. $R^5$ is $SO_2NR^6R^7$.
b. $R^3$ is $C_1$-$C_6$ alkyl.
2b1. $R^5$ is $C_1$-$C_6$ alkyl.
2b2. $R^5$ is $SO_2NR^6R^7$.
c. $R^3$ is hydroxy.
2c1. $R^5$ is $C_1$-$C_6$ alkyl.
2c2. $R^5$ is $SO_2NR^6R^7$.

3. $R^1$ is $C_2$-$C_6$ alkynyl.
a. $R^3$ is hydrogen.
3a1. $R^5$ is $C_1$-$C_6$ alkyl.
3a2. $R^5$ is $SO_2NR^6R^7$.
b. $R^3$ is $C_1$-$C_6$ alkyl.
3b1. $R^5$ is $C_1$-$C_6$ alkyl.
3b2. $R^5$ is $SO_2NR^6R^7$.

4. $R^1$ is halo-$C_1$-$C_6$ alkyl.
a. $R^3$ is hydrogen.
4a1. $R^5$ is $C_1$-$C_6$ alkyl.
4a2. $R^5$ is $SO_2NR^6R^7$.
b. $R^3$ is $C_1$-$C_6$ alkyl.
4b1. $R^5$ is $C_1$-$C_6$ alkyl.
4b2. $R^5$ is $SO_2NR^6R^7$.
c. $R^3$ is hydroxy.
4c1. $R^5$ is $C_1$-$C_6$ alkyl.
4c2. $R^5$ is $SO_2NR^6R^7$.

B. Those of the formula (V):

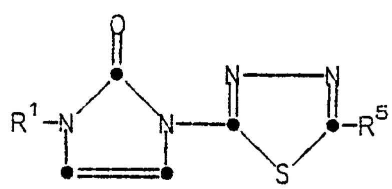

(V)

1. $R^1$ is $C_1$-$C_6$ alkyl.
a. $R^5$ is $C_1$-$C_6$ alkyl.
1a1. $R^5$ is <u>tert</u>.-butyl.
1a2. $R^1$ is methyl.
1b. $R^5$ is halo-$C_1$-$C_6$ alkyl.
1c. $R^5$ is $C_2$-$C_6$ alkenyl.
1d. $R^5$ is $C_2$-$C_6$ alkynyl.
1e. $R^5$ is $C_1$-$C_6$ alkylthio.
1f. $R^5$ is $C_1$-$C_6$ alkylsulfinyl.
1g. $R^5$ is $C_1$-$C_6$ alkylsulfonyl.
1h. $R^5$ is $SO_2NR^6R^7$.
1i. $R^5$ is $SO_2NR^6R^7$ where $R^6$ and $R^7$ independently are $C_1$-$C_6$ alkyl.

2. $R^1$ is $C_2$-$C_6$ alkenyl.
a. $R^5$ is $C_1$-$C_6$ alkyl.
2a1. $R^5$ is <u>tert</u>.-butyl.
2b. $R^5$ is $C_2$-$C_6$ alkenyl.
2c. $R^5$ is $C_2$-$C_6$ alkynyl.
2d. $R^5$ is $SO_2NR^6R^7$.

3. $R^1$ is $C_2$-$C_6$ alkynyl.
a. $R^5$ is $C_1$-$C_6$ alkyl.
3a1. $R^5$ is <u>tert</u>.-butyl.
2b. $R^5$ is $C_2$-$C_6$ alkenyl.
2c. $R^5$ is $C_2$-$C_6$ alkynyl.
2d. $R^5$ is $SO_2NR^6R^7$.

C. Those of the formula (VI):

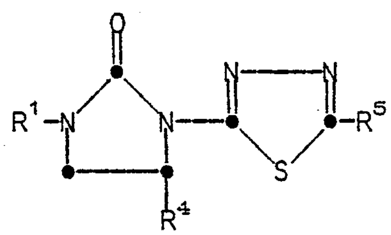

(VI)

1. $R^4$ is $C_1$-$C_6$ alkanoyloxy.
a. $R^1$ is $C_1$-$C_6$ alkyl.
1a1. $R^1$ is methyl.
1a2. $R^5$ is $C_1$-$C_6$ alkyl.
1a3. $R^5$ is <u>tert</u>.-butyl.
1a4. $R^5$ is $C_2$-$C_6$ alkenyl.
1a5. $R^5$ is $C_2$-$C_6$ alkynyl.
1a6. $R^5$ is $SO_2NR^6R^7$.
b. $R^1$ is $C_2$-$C_6$ alkenyl.
1b1. $R^5$ is $C_1$-$C_6$ alkyl.

1b2. $R^5$ is $SO_2NR^6R^7$.

c. $R^1$ is $C_2$-$C_6$ alkynyl.

1c1. $R^5$ is $C_1$-$C_6$ alkyl.

1c2. $R^5$ is $SO_2NR^6R^7$.

All of the compounds employed in the method of this invention, except those wherein $R^5$ is $SO_2NR^6R^7$, are known in the art and are readily available by art known methods. Those compounds of the above formula wherein $R^5$ is $SO_2NR^6R^7$ are new, and the preparation of typical compounds is exemplified below.

Example 1

3-(5-Dimethylaminosulfonyl-1,3,4-thiadiazol-2-yl)-4-hydroxy-1-methyl-2-imidazolidinone

A. Preparation of 2-(N-phenoxycarbonylamino)-1,3,4-thiadiazol-5-thiol.

To a stirred solution of 25 g (0.19 moles) of 2-amino-1,3,4-thiadiazol-5-thiol in 75 ml of pyridine were added dropwise over 30 minutes 32.8 g (0.21 moles) of phenyl chloroformate. Following complete addition the reaction mixture was warmed to room temperature and stirred for 16 hours. The reaction mixture was then poured into ice and the pH was adjusted to 2.0 by the addition of hydrochloric acid. The solid which precipitated was collected by filtration and washed with water and air dried to provide 48 g of 2-(N-phenoxycarbonylamino)-1,3,4-thiadiazol-5-thiol. Melting point 203-207°C.

B. Preparation of 2-(N-phenoxycarbonylamino)-5-dimethylaminosulfonyl-1,3,4-thiadiazole.

To a cold (5-10°C.) stirred suspension of

2-(N-phenoxycarbonylamino)-1,3,4-thiadiazol-5-thiol (48 g) in 60 ml of 12N hydrochloric acid and 200 ml of water was bubbled chlorine until chlorine was no longer absorbed. The precipitate that formed was filtered and washed with water to provide 51 g of 2-(N-phenoxycarbonylamino)-5-chlorosulfonyl-1,3,4-thiadiazole. mp 166-169°C. The product thus formed was added portionwise over 30 minutes to a stirred mixture of 60 ml of 25% aqueous dimethylamine and 30 ml of water. The reaction mixture was stirred overnight at room temperature following complete addition. The reaction mixture next was filtered and the precipitate was washed with water and air dried to give 17 g of 2-(N-phenoxycarbonylamino)-5-dimethylaminosulfonyl-1,3,4-thiadiazole. mp 155-159°C.

C. Preparation of 1-(5-Dimethylaminosulfonyl-1,3,4-thiadiazol-2-yl)-3-(2,2-dimethoxyethyl)-3-methylurea.

A suspension of 25 g (0.076 moles) of 2-(N-phenoxycarbonylamino)-5-dimethylaminosulfonyl-1,3,4-thiadiazole in 100 ml of benzene containing 10 g (0.084 moles) of N-methyl-(2,2-dimethoxy)ethylamine was heated at reflux for 12 hours. The reaction mixture was cooled to room temperature and the solvent was removed by evaporation under reduced pressure to provide 23 g of a dark oil. The oil was shown to be a mixture of 1-(5-dimethylaminosulfonyl-1,3,4-thiadiazol-2-yl)-3-(2,2-dimethoxyethyl)-3-methylurea and phenol.

D. A suspension of the oil in 160 ml of water containing 4 ml of concentrated hydrochloric

acid was heated at reflux for 30 minutes. The reaction mixture was then cooled to room temperature and stirred for 12 hours. The precipitate that had formed was filtered and washed with water and then was crystallized from ethyl acetate and recrystallized from ethanol to provide 7.4 g of 3-(5-dimethylaminosulfonyl-1,3,4-thiadiazol-2-yl)-4-hydroxy-1-methyl-2-imidazolidinone. mp 194-196°C.

Example 2

3-(5-Diethylaminosulfonyl-1,3,4-thiadiazol-2-yl)-4-hydroxy-1-methyl-2-imidazolidinone

A. Following the general procedures set forth in Example 1, 3.9 g (0.025 moles) of phenylchloroformate were added dropwise over 30 minutes to a stirred solution of 5.4 g (0.023 moles) of 2-amino-5-diethylaminosulfonyl-1,3,4-thiadiazole in 40 ml of pyridine. The reaction mixture was stirred at about 10°C. during the addition of the phenylchloroformate, and following the addition the reaction mixture was warmed to about 20°C. and stirred at that temperature for about 3 hours. The reaction mixture was then added to 50 g of ice and diluted with one normal hydrochloric acid to pH 2.0. The precipitated solid was collected by filtration and washed with water and air dried to give 10.8 g of 2-(N-phenoxycarbonylamino)-5-diethylaminosulfonyl-1,3,4-thiadiazole. mp 126-130°C.

B. A suspension of 10.8 g. of the thiadiazole-carbamate prepared as described above in 100 ml. of toluene was stirred while 3.9 g (0.033 mole) of N-methyl-(2,2-dimethoxy)ethylamine were added dropwise

over 20 minutes. The reaction mixture was then heated at 80°C for 2 hours and then cooled to room temperature and the solvent was removed by evaporation under reduced pressure to provide 6.3 g of the product as an oil. The oil solidified on standing and the solid was crystallized from ethyl acetate and hexane to give 1-(5-diethylaminosulfonyl-1,3,4-thiadiazol-2-yl)-3-(2,2-dimethoxyethyl)-3-methylurea. mp 110-112°C.

Analysis calculated for $C_{12}H_{23}N_5O_5S_2$

Theory: C, 37.78; H, 6.08; N, 18.36.

Found: C, 37.76; H, 5.82; N, 18.12.

C. A solution of 4.3 g of the thiadiazolylurea thus prepared was suspended in 80 ml of water containing 2 ml of concentrated hydrochloric acid. The reaction mixture was heated at reflux for 45 minutes and then cooled to room temperature and added to 50 g of ice. The product crystallized out of the aqueous mixture and was collected by filtration and recrystallized from ethyl acetate and hexane to give 2.0 g of 3-(5-diethylaminosulfonyl-1,3,4-thiadiazol-2-yl)-4-hydroxy-1-methyl-2-imidazolidinone. mp 161-163°C.

Analysis calculated for $C_{10}H_{17}N_5O_4S_2$

Theory: C, 35.82; H, 5.07; N, 20.90.

Found: C, 36.05; H, 4.85; N, 20.94.

Example 3

3-(5-N-methyl-N-ethylaminosulfonyl-1,3,4-thiadiazol-2-yl)-4-hydroxy-1-methyl-2-imidazolidinone

A. Following the general procedures set forth in Examples 1 and 2, 11.5 g (0.033 moles) of 2-phenoxy-

carbonylamino-5-(N-methyl-N-ethylaminosulfonyl)-1,3,4-thiadiazole were reacted with 4.3 g (0.036 moles) of N-methyl-(2,2-dimethoxy)ethylamine in 100 ml. of benzene to give 14 g of 1-(5-N-methyl-N-ethylaminosulfonyl-1,3,4-thiadiazol-2-yl)-3-(2,2-dimethoxyethyl)-3-methylurea.

B. The urea thus formed was reacted with hydrochloric acid in water to provide 1.72 g of 3-(5-N-methyl-N-ethylaminosulfonyl-1,3,4-thiadiazol-2-yl)-4-hydroxy-1-methyl-2-imidazolidinone. mp 130-132°C.

Analysis calculated for $C_9H_{15}N_5O_4S_2$

Theory: C, 33.64; H, 4.67; N, 21.81.

Found: C, 33.63; H, 4.51; N, 21.55.

Example 4

3-(5-N-methyl-N-propylaminosulfonyl-1,3,4-thiadiazol-2-yl)-4-hydroxy-1-methyl-2-imidazolidinone

A. Following the general procedure set forth in Examples 1 and 2, 14.3 g (0.04 moles) of 2-phenoxycarbonylamino-5-(N-methyl-N-propylaminosulfonyl)-1,3,4-thiadiazole were reacted with 5.2 g (0.044 moles) of N-methyl-(2,2-dimethoxy)ethylamine in refluxing benzene to give 13.2 g of 3-(5-N-methyl-N-propylaminosulfonyl-1,3,4-thiadiazol-2-yl)-1-(2,2-dimethoxyethyl)-1-methylurea as an oil.

B. The thiadiazolylurea thus prepared, 13.2 g (0.035 moles), was reacted with 2 ml of concentrated hydrochloric acid and 80 ml of water at about 100°C. for 30 minutes. The reaction mixture was cooled to room temperature and added to ice. The product was extracted into ethyl acetate and the organic extracts were combined, washed with water, dried and the solvent

was removed by evaporation under reduced pressure to provide, after recrystallization from ethyl acetate and hexane, 1.45 g of 3-(5-N-methyl-N-propylaminosulfonyl-1,3,4-thiadiazol-2-yl)-4-hydroxy-1-methyl-2-imidazolidinone. mp 126-128°C.

Analysis calculated for $C_{10}H_{17}N_5O_4S_2$
Theory: C, 35.81; H, 5.11; N, 20.88.
Found: C, 35.99; H, 4.93; N, 20.80.

Example 5

3-[5-N-Methyl-N-(n-butyl)aminosulfonyl-1,3,4-thiadiazol-2-yl]-4-hydroxy-1-methyl-2-imidazolidinone

A. 5-Amino-1,3,4-thiadiazol-2-thiol was reacted with chlorine in an aqueous solution of hydrochloric acid to provide 2-amino-5-chlorosulfonyl-1,3,4-thiadiazole.

B. The product of Step A was reacted with N-methyl-n-butylamine in the presence of triethylamine in tetrahydrofuran to provide 2-amino-5-[N-methyl-N-(n-butyl)aminosulfonyl]-1,3,4-thiadiazole.

C. Reaction of the compound produced in Step B with phenyl chloroformate according to the procedure of Example 1 provided 2-phenoxycarbonylamino-5-[N-methyl-N-(n-butyl)aminosulfonyl]-1,3,4-thiadiazole.

D. To a stirred solution of 13.7 g (0.037 mole) of 2-phenoxycarbonylamino-5-[N-methyl-N-(n-butyl-aminosulfonyl]-1,3,4-thiadiazole in 100 ml of benzene were added dropwise over 30 minutes 4.9 g (0.04 mole) of N-methyl-(2,2-dimethoxy)ethylamine. The reaction mixture was heated at reflux for 3 hours and then cooled to room temperature. The solid precipitate was

collected by filtration to provide 6.9 g of 1-[5-N-methyl-N-(n-butyl)aminosulfonyl-1,3,4-thiadiazol-2-yl]-3-(2,2-dimethoxy)ethyl-3-methylurea. A sample which was recrystallized from ethanol and water melted at 95-97°C.

Analysis calculated for $C_{13}H_{25}N_5O_5S_2$
Theory: C, 39.48; H, 6.37; N, 17.71.
Found: C, 39.72; H, 6.13; N, 17.50.

E. A solution of the thiadiazolylurea in 80 ml of water containing 2 ml of concentrated hydrochloric acid was heated at reflux for 45 minutes. The reaction mixture was then cooled and added to 150 g of ice. The aqueous mixture was extracted several times with ethyl acetate, and the organic extracts were combined, washed with water, dried and the solvent was removed by evaporation under reduced pressure to provide the product as an oil. The oil was crystallized from ethyl acetate and hexane to afford 1.5 g of 3-[5-N-methyl-N-(n-butyl)aminosulfonyl-1,3,4-thiadiazol-2-yl]-4-hydroxy-1-methyl-2-imidazolidinone. mp 105-107°C.

Analysis calculated for $C_{11}H_{19}N_5O_4S_2$
Theory: C, 37.81; H, 5.48; N, 20.04.
Found: C, 37.52; H, 5.28; N, 19.84.

## Example 6

3-[5-N-Methyl-N-(2-propynyl)aminosulfonyl-1,3,4-thiadiazol-2-yl]-4-hydroxy-1-methyl-2-imidazolidinone

A. N-methylpropargylamine was reacted with 2-amino-5-chlorosulfonyl-1,3,4-thiadiazole to provide

2-amino-5-[N-methyl-N-(2-propynyl)aminosulfonyl]-1,3,4-thiadiazole.

B. The aminothiadiazole derivative of Step A was reacted with phenyl chloroformate to give 2-phenoxycarbonylamino-5-[N-methyl-N-(2-propynyl)aminosulfonyl]-1,3,4-thiadiazole.

C. A solution of 12 g (0.034 mole) of 2-phenoxycarbonylamino-5-[N-methyl-N-(2-propynyl)aminosulfonyl]-1,3,4-thiadiazole in 100 ml of benzene containing 4.4 g (0.037 mole) of N-methyl-(2,2-dimethoxy)ethylamine was heated at reflux for 3 hours and then cooled to room temperature. The reaction solvent was removed by evaporation under reduced pressure to provide 11.0 g of 1-[5-N-methyl-N-(2-propynyl)aminosulfonyl-1,3,4-thiadiazol-2-yl]-3-(2,2-dimethoxyethyl)-3-methylurea. A portion of the product was recrystallized from ethanol and water and melted at 107-109°C.

Analysis calculated for $C_{12}H_{19}N_5O_5S_2$

Theory: C, 38.19; H, 5.07; N, 18.56.

Found: C, 37.98; H, 4.86, N, 18.31.

D. Cyclization of 8.0 g of this thiadiazolylurea derivative by reaction with hydrochloric acid and water according to the procedure outlined above in Example 1 provided 3.6 g of 3-[5-N-methyl-N-(2-propynyl)-aminosulfonyl-1,3,4-thiadiazol-2-yl]-4-hydroxy-1-methyl-2-imidazolidinone. mp 146-148°C.

Analysis calculated for $C_{10}H_{13}N_5O_4S_2$

Theory: C, 36.25; H, 3.95; N, 21.13.

Found: C, 36.08; H, 3.69; N, 20.93.

## Example 7

3-(5-N-Methyl-N-methoxyaminosulfonyl-1,3,4-thiadiazol-2-yl)-4-hydroxy-1-methyl-2-imidazolidinone

A. By following the general procedure outlined above, N-methylmethoxyamine was reacted with 2-amino-5-chlorosulfonyl-1,3,4-thiadiazole to give 2-amino-5-N-methyl-N-methoxyaminosulfonyl-1,3,4-thiadiazole.

B. The product of Step A was reacted with phenyl chloroformate to give the corresponding phenylcarbamate derivative.

C. The product of Step B was then reacted with N-methyl-(2,2-dimethoxy)ethylamine to provide 1-(5-N-methyl-N-methoxyaminosulfonyl-1,3,4-thiadiazol-2-yl)-3-(2,2-dimethoxyethyl)-3-methylurea. mp 134-136°C.

Analysis calculated for $C_{10}H_{19}N_5O_6S_2$

Theory: C, 32.51; H, 5.18; N, 18.96.

Found: C, 32.57; H, 5.16; N, 19.04.

D. Cyclization of 4.0 g of the thiadiazolylurea derivative of Step C by reaction with hydrochloric acid and water provided, following crystallization from ethyl acetate, 2.03 g of 3-(5-N-methyl-N-methoxyaminosulfonyl-1,3,4-thiadiazol-2-yl)-4-hydroxy-1-methyl-2-imidazolidinone. mp 178-180°C.

Analysis calculated for $C_8H_{13}N_5O_5S_2$

Theory: C, 29.72; H, 4.05; N, 21.66.

Found: C, 29.75; H, 4.15; N, 21.86.

Example 8

3-(5-Dimethylaminosulfonyl-1,3,4-thiadiazol-2-yl)-1-methyl-2-imidazolinone

A solution of 3 g of 3-(5-dimethylaminosulfonyl-1,3,4-thiadiazol-2-yl)-4-hydroxy-1-methyl-2-imidazolidinone (prepared as described in Example 1) in 20 g of acetyl chloride was stirred at 24°C. for 72 hours. The reaction mixture was then concentrated by evaporation of the acetyl chloride under reduced pressure and the residue was crystallized from methanol and again from ethyl acetate to provide 0.5 g of 3-(5-dimethylaminosulfonyl-1,3,4-thiadiazol-2-yl)-1-methyl-2-imidazolinone. mp 192-195°C.

Analysis calculated for $C_8H_{11}N_5O_3S_2$

Theory: C, 33.21; H, 3.83; N, 24.21.

Found: C, 33.46; H, 3.81; N, 24.00.

Example 9

3-(5-Dimethylaminosulfonyl-1,3,4-thiadiazol-2-yl)-4-acetoxy-1-methyl-2-imidazolidinone

A solution of 1 g (0.003 mole) of 3-(5-dimethylaminosulfonyl-1,3,4-thiadiazol-2-yl)-4-hydroxy-1-methyl-2-imidazolidinone (from Example 1) in 10 ml of dichloromethane was cooled to 0°C. and stirred. To the cold stirred reaction mixture were added 0.31 g (0.004 mole) of pyridine followed by the dropwise addition of 0.31 g (0.004 mole) of acetyl chloride over 20 minutes. The reaction mixture was stirred at 0°C. for 5 hours following complete addition. The mixture was then filtered and the filtrate was reduced in volume by

evaporation of the solvent under reduced pressure to give an oil. The oil was added to 50 ml of water and the aqueous mixture was diluted with 1N hydrochloric acid to pH 7. The aqueous mixture was extracted several times with ethyl acetate and the extracts were combined, washed with water, dried and the solvent was removed by evaporation under reduced pressure. The product was crystallized once from ethanol and again from ethyl acetate to afford 0.4 g of 3-(5-dimethylaminosulfonyl-1,3,4-thiadiazol-2-yl)-4-acetoxy-1-methyl-2-imidazolidinone. mp 163-165°C. Analysis calculated for $C_{10}H_{15}N_5O_5S_2$

Theory: C, 34.38; H, 4.33; N, 20.05.

Found: C, 34.66; H, 4.35; N, 20.04.

## Example 10

3-(5-Dimethylaminosulfonyl-1,3,4-thiadiazol-2-yl)-4-methoxy-1-methyl-2-imidazolidinone

A solution of 2.0 g of 3-(5-dimethylaminosulfonyl-1,3,4-thiadiazol-2-yl)-4-hydroxy-1-methyl-2-imidazolidinone (prepared according to the method of Example 1) in 15 ml of methanol was stirred at room temperature while 5 drops of concentrated sulfuric acid were added. The reaction mixture was stirred at room temperature for 2 hours and then heated to reflux for 12 hours. The mixture was cooled to room temperature and then filtered. The precipitate was washed with fresh methanol. The filtrate was concentrated to dryness by evaporation of the solvent under reduced pressure and the solid thus formed was crystallized

from ethanol to give 0.4 g of 3-(5-dimethylaminosulfonyl-1,3,4-thiadiazol-2-yl)-4-methoxy-1-methyl-2-imidazolidinone. mp 129-131°C. Analysis calculated for $C_9H_{15}N_5O_4S_2$

Theory: C, 33.64; H, 4.70; N, 21.79.
Found: C, 33.54; H, 4.81; N, 21.56.

Example 11

3-(5-Dimethylaminosulfonyl-1,3,4-thiadiazol-2-yl)-4-hydroxy-2-imidazolidinone

A. To a stirred solution of 12.0 g (0.036 mole) of 2-phenoxycarbonylamino-5-dimethylaminosulfonyl-1,3,4-thiadiazole in 100 ml of toluene were added dropwise over 20 minutes 4.2 g (0.040 mole) of 2,2-dimethoxyethylamine. The reaction mixture was heated at 80° for 2 hours and then cooled and concentrated by removal of the reaction solvent by evaporation under reduced pressure. The product thus formed was an oil that solidified on standing. Recrystallization from ethanol afforded 4.7 g of 1-(5-dimethylaminosulfonyl-1,3,4-thiadiazol-2-yl)-3-(2,2-dimethoxyethyl)urea. Analysis calculated for $C_9H_{17}N_5O_5S_2$

Theory: C, 31.85; H, 5.05; N, 20.64.
Found: C, 32.14; H, 5.18; N, 20.78.

mp 172-174°C.

B. A solution of 4 g of 1-(5-dimethylaminosulfonyl)-1,3,4-thiadiazol-2-yl)-3-(2,2-dimethoxyethyl)urea in 80 ml of water containing 2 ml of concentrated hydrochloric acid was stirred at reflux for

30 minutes. The mixture was then cooled and the product was collected by filtration. The solid product was washed with fresh water and air dried to give 2.2 g of 3-(5-dimethylaminosulfonyl-1,3,4-thiadiazol-2-yl)-4-hydroxy-2-imidazolidinone. mp 204-206°C.

Analysis calculated for $C_7H_{11}N_5O_4S_2$

Theory: C, 28.66; H, 3.78; N, 23.88.

Found: C, 28.53; H, 3.67; N, 23.68.

## Example 12

3-(5-Dimethylaminosulfonyl-1,3,4-thiadiazol-2-yl)-4-hydroxy-1-phenyl-2-imidazolidinone

A. To a stirred solution of 5.0 g of 2-phenoxycarbonylamino-5-dimethylaminosulfonyl-1,3,4-thiadiazole in 30 ml. of tetrahydrofuran were added dropwise over 30 minutes 3.6 g of N-phenyl-N-(2,2-dimethoxyethyl)-amine. Following complete addition, 1 ml of pyridine was added to the reaction mixture and the mixture was heated at reflux for 16 hours. The reaction mixture was then cooled to room temperature and concentrated in volume by removal of the reaction solvent by evaporation under reduced pressure. The residue was poured into 20 ml of ice water and the pH of the aqueous mixture was adjusted to 5 by the addition of dilute hydrochloric acid. The aqueous acid mixture was extracted several times with ethyl acetate and the extracts were combined, washed with water and dried. Removal of the solvent by evaporation under reduced pressure provided the product as an oil. The oil was purified by column chromatography over silica gel,

eluting with diethyl ether saturated with water. Removal of the solvent from the appropriate fractions afforded 2.2 g of 3-(5-dimethylaminosulfonyl-1,3,4-thiadiazol-2-yl)-3-phenyl-3-(2,2-dimethoxyethyl)urea. mp 59-61°C. Analysis calculated for $C_{15}H_{21}N_5O_5S_2$

Theory: C, 43.36; H, 5.09; N, 16.86.
Found: C, 43.59; H, 5.02; N, 16.96.

B. The thiadiazolylurea thus formed (1.8 g) was cyclized by reaction with 1 ml of concentrated hydrochloric acid in 40 ml of water. The reaction mixture was heated at reflux for 30 minutes and then cooled and extracted with ethyl acetate. The combined organic extracts were washed with water, dried, and the solvent was removed by evaporation under reduced pressure. The solid that was formed was crystallized from ethanol and water and again from ethyl acetate to afford 180 mg of 3-(5-dimethylaminosulfonyl-1,3,4-thiadiazol-2-yl)-4-hydroxy-1-phenyl-2-imidazolidinone. mp 179-181°C. Analysis calculated for $C_{13}H_{15}N_5O_4S_2$

Theory: C, 42.28; H, 4.07; N, 18.97.
Found: C, 42.11; H, 3.85; N, 18.82.

Example 13

3-(5-Dimethylaminosulfonyl-1,3,4-thiadiazol-2-yl)-4-methylaminocarbonyloxy-1-methyl-2-imidazolidinone

To a stirred suspension of 2.0 g (0.007 mole) of 3-(5-dimethylaminosulfonyl-1,3,4-thiadiazol-2-yl)-4-hydroxy-1-methyl-2-imidazolidinone (prepared as

described in Example 1) in 15 ml of toluene were added dropwise 0.49 ml (0.008 mole) of methyl isocyanate. Following complete addition, the reaction mixture was heated at reflux for 16 hours. The reaction mixture was filtered while hot, and the filtrate was then cooled to room temperature. The solvent was removed by evaporation under reduced pressure to provide the product as a solid. The solid was recrystallized from ethanol to afford 0.66 g of 3-(5-dimethylaminosulfonyl-1,3,4-thiadiazol-2-yl)-4-methylaminocarbonyloxy-1-methyl-2-imidazolidinone. mp 190-192°C. Analysis calculated for $C_{10}H_{16}N_6O_5S_2$

Theory: C, 32.96; H, 4.43; N, 23.06.
Found: C, 32.90; H, 4.55; N, 22.86.

## Example 14

Following the general procedure of Example 13 1 g of 3-(5-dimethylaminosulfonyl-1,3,4-thiadiazol-2-yl)-4-hydroxy-1-methyl-2-imidazolidinone from Example 1 was reacted with 0.48 g of phenyl isocyanate to provide, following crystallization from acetone, 0.3 g of 3-(5-dimethylaminosulfonyl-1,3,4-thiadiazol-2-yl)-4-phenylaminocarbonyloxy-1-methyl-2-imidazolidinone. mp 221-223°C. Analysis calculated for $C_{15}H_{18}N_6O_5S_2$

Theory: C, 42.25; H, 4.25; N, 19.71.
Found: C, 42.02; H, 4.08; N, 19.51.

Example 15

3-(5-Dimethylaminosulfonyl-1,3,4-thiadiazol-2-yl)-4-dimethylamino-1-methyl-2-imidazolidinone

Two grams of 3-(5-dimethylaminosulfonyl-1,3,4-thiadiazol-2-yl)-4-hydroxy-1-methyl-2-imidazolidinone (prepared as described in Example 1) were suspended in 10 ml of THF. To the stirred reaction mixture were added 1.14 g of dimethylamine hydrochloride and 1.42 g of triethylamine. The reaction mixture was heated at 60°C for 7 hours and then cooled to room temperature and stirred for an additional 16 hours. Thin layer chromatographic analysis indicated that the reaction mixture was comprised of approximately 50% starting material. The reaction mixture was therefore heated at 60°C for an additional seven and one-half hours, and then stirred for 12 hours at room temperature. The solvent was removed from the reaction mixture by evaporation under reduced pressure to provide the product as an oil. The oil was dissolved in 100 ml of ethyl acetate and washed with water, dried, and the solvent was removed by evaporation. The product thus formed was purified by column chromatography over silica gel, using ethyl acetate as eluant. Evaporation of the solvent from the appropriate fractions provided 1.25 g of 3-(5-dimethylaminosulfonyl-1,3,4-thiadiazol-2-yl)-4-dimethylamino-1-methyl-2-imidazolidinone. mp 98-100°C. Analysis calculated for $C_{10}H_{18}N_6O_3S_2$

Theory: C, 35.92; H, 5.43; N, 25.13.
Found: C, 35.66; H, 5.22; N, 24.96.

Example 16

3-(5-Dimethylaminosulfonyl-1,3,4-thiadiazol-2-yl)-4-hydroxy-1-methyl-2-imidazolidinone

This Example is a preferred procedure for the synthesis of the compound of Example 1.

A. Into a stirred suspension of 50 g (0.38 mole) of 2-amino-1,3,4-thiadiazol-5-thiol in 200 ml of water containing 60 ml of concentrated hydrochloric acid was bubbled chlorine until the suspension became saturated and chlorine was no longer absorbed. The reaction mixture was then filtered and the brown solid was identified as 56 g of 2-amino-5-chlorosulfonyl-1,3,4-thiadiazole. mp 127-130°C.

B. The 2-amino-5-chlorosulfonyl-1,3,4-thiadiazole was suspended in 200 ml of water and stirred at 0-10°C while 140 ml of 25% (w/w) aqueous dimethylamine were added dropwise over thirty minutes. The reaction mixture was warmed to room temperature following the addition, and stirring was continued for one hour. The solid was collected by filtration and air dried to give 46 g of 2-amino-5-dimethylaminosulfonyl-1,3,4-thiadiazole. mp 152-155°C.

C. To a cold (0-5°C) stirred solution of 20.0 g (0.096 mole) of 2-amino-5-dimethylaminosulfonyl-1,3,4-thiadiazole in 75 ml of pyridine were added 17.2 g of phenyl chloroformate dropwise over thirty minutes. The reaction mixture was then warmed to room temperature and stirred for an additional two and one-half hours. The reaction mixture was next added to 200 g of ice water and acidified to pH 2 by the addition of hydrochloric acid. The precipitated solid was

collected by filtration, washed with water, dried, and identified as 31.3 g of 2-(N-phenoxycarbonylamino)-5-dimethylaminosulfonyl-1,3,4-thiadiazole. mp 186-190°C.

D. The above procedure was repeated several times to produce 1012 g of 2-(N-phenoxycarbonylamino)-5-dimethylaminosulfonyl-1,3,4-thiadiazole. This entire lot was then suspended in 2800 ml of benzene, and the suspension was stirred at room temperature while 405 g of N-methyl-(2,2-dimethoxy)ethylamine were added dropwise over two hours. Following the addition, the reaction mixture was heated at reflux for two hours. The reaction mixture was next cooled to room temperature and the solvent was removed by evaporation under reduced pressure to give 1010 g of 1-(5-dimethylaminosulfonyl-1,3,4-thiadiazol-2-yl)-3-(2,2-dimethoxyethyl)-3-methylurea (containing slight amount of phenol impurity). mp 120-124°C.

E. A suspension of 1010 g of the urea thus prepared in 6000 ml of water containing 150 ml of concentrated hydrochloric acid was heated at reflux for thirty minutes. The reaction mixture was then cooled and filtered. The filter cake was washed with fresh water and then air dried. The solid was next slurried in 8 liters of ethanol, filtered, and then washed with diethyl ether and air dried to give 691 g of 3-(5-dimethylaminosulfonyl-1,3,4-thiadiazol-2-yl)-4-hydroxy-1-methyl-2-imidazolidinone. mp 194-196°C.

Analysis calculated for $C_8H_{13}N_5O_4S_2$

Theory: C, 31.26; H, 4.26; N, 22.79.

Found: C, 31.13; H, 4.01; N, 22.56.

The aquatic method provided by this invention permits the control and elimination of aquatic weeds employing a compound defined herein. The invention contemplates the control and elimination of all forms of plant life that infests ponds, lakes, water ways, water reservoirs, including water towers and containers employed in the storage of water for human consumption. The invention thus provides a method for controlling algae and aquatic plant growth including submersed, floating, emergent and ditchbank vegetation. Because the compounds are useful in controlling aquatic plant life, an important embodiment of this invention is a method of use employing the compounds.

The compounds defined by this invention are particularly useful as aquatic algicides, aquatic growth regulators and aquatic herbicides. A preferred embodiment of this invention is a method for controlling aquatic plant growth which comprises applying an aquatic herbicidally effective amount of a compound as defined herein to the aquatic plants to be controlled or to the water in which the plants are growing.

The compounds of the invention have been evaluated in a standard aquatic algicide test designed to show algicidal activity. In a primary screen, the compounds were evaluated against Chlorella vulgaris, Scenedesmus quadricanda, and Anacystis nidulans. These algae species were grown on agar slants containing artificial media (Hughes' media). The agar slants were employed in the inoculation of the test media, which itself is an aqueous Hughes' media. Five milliliters of sterile Hughes' media is used to wash

the agar slants, and this is then added to 400 ml of sterile Hughes' media. Two milliliters of the inoculated media is then added to each of several 12 ml disposable vials.

The test compounds are formulated for evaluation by dissolving 10 mg of compound in a solution of 0.5 ml acetone and 4.5 ml of sterile 0.1% aqueous polyoxyethylene sorbitan monooleate (Tween 80). Aliquot portions of the formulated test compounds are then added to the vials containing the various algae species. Visual observations and comparisons to non-treated control vials were made 7 days after treatment. Activity ratings were made on a scale of 1 to 5 according to the following meanings:

1 = no effect
2 = slight effect
3 = moderate effect
4 = heavy effect
5 = 100% control.

Table 1 which follows presents the algicidal activity of compounds of the invention evaluated according to the foregoing procedure.

Table 1

Aquatic Algicide Activity

| Compound Evaluated | Concentration ppm | Control Ratings Chlorella vulgaris | Scenedesmus quadricanda | Anacystis nidulans |
|---|---|---|---|---|
| Control | | 0 | 0 | 0 |
| 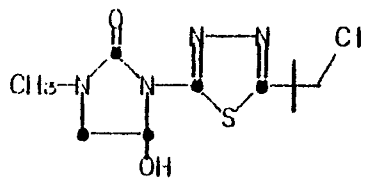 | 10 | 5 | 4 | 5 |
| 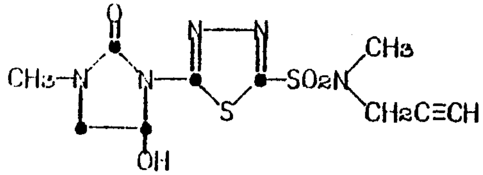 | 10 | 4 | 3 | 5 |
| 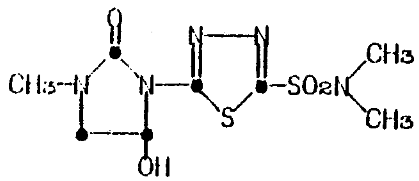 | 10 | 5 | 5 | 5 |
| 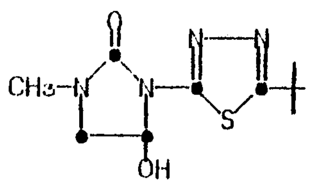 | 10 | 5 | 1 | 5 |

Table 1 continued

| Compound Evaluated | Concentration ppm | Control Ratings Chlorella vulgaris | Scenedesmus quadricanda | Anacystis nidulans |
|---|---|---|---|---|
| 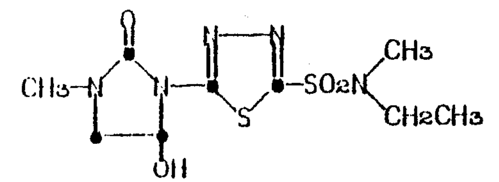 | 10 | 4 | 4 | 4 |
| | 1 | 5 | 4 | 5 |
| | 0.5 | 5 | 4 | 5 |
| 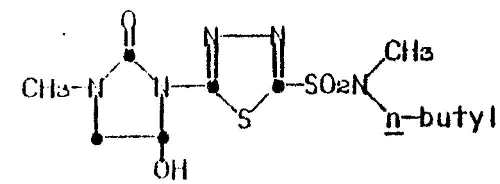 | 10 | 5 | 4 | 5 |
| 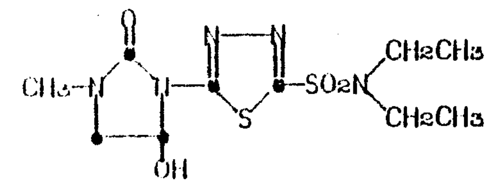 | 10 | 5 | 5 | 5 |
| | 1 | 5 | 4 | 5 |
| | 0.5 | 5 | 4 | 5 |
| 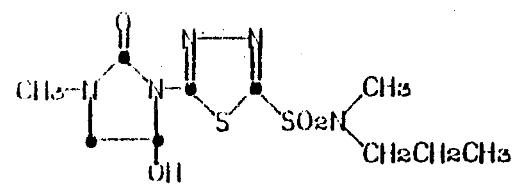 | 10 | 5 | 5 | 5 |
| | 1 | 3 | 1 | - |
| | 0.5 | 3 | 1 | - |

Table 1 continued

| Compound Evaluated | Concentration ppm | Control Ratings Chlorella vulgaris | Scenedesmus quadricanda | Anacystis nidulans |
|---|---|---|---|---|
| 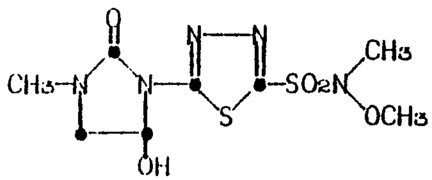 | 10 | 5 | 4 | 5 |
| 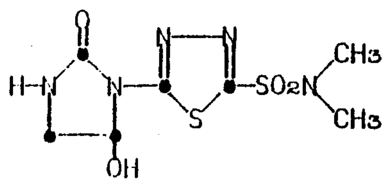 | 10 | 3 | 4 | 1 |

As noted above, the compounds of this invention also have activity in the regulation of aquatic plant growth and are useful as aquatic herbicides against plants such as naiad, watergrass, cat-tail, duckweed, parrot-feathers, smartweed, water primrose, chara, milfoil and related aquatic vegetation. The following method was used in the laboratory to evaluate the aquatic growth regulating properties of the compounds disclosed herein.

The compounds for this test were formulated in the following manner. Twenty milligrams of compound was weighed into a 12 ml disposable vial. To the vial containing the compound were added 1 ml of acetone and 9 ml of aqueous 0.1 percent polyoxyethylene sorbitan monooleate (Tween 80). This solution was then diluted with appropriate volumes of water to obtain solutions containing 10, 1, 0.5 and 0.25 ppm (parts per million) of test compound.

Terminal pieces of aquatic plants such as Florida elodea, Hydrilla verticillata (L.F.), (hereinafter identified as hydrilla) 10 cm long, without branching, were prepared for testing. Three such cuttings were placed in each plastic container holding 785 ml of water containing the formulated test compound and 3 ml of Hoagland's nutrient solution. Three 10 cm cuttings of hydrilla were placed in each of several control containers of water. To the water in each control container there was also added the amount of solvent used to formulate the test compound for each container.

After a period of two to three weeks, measurements were made to determine the total length of each

plant. An average total growth was obtained by dividing the total combined lengths by the number of replicates. By subtracting 10 cm from the average total length, the average increase in growth was obtained. This difference was divided by the average increase in length of the plants in the solvent controls (SC) and the quotient multiplied by 100 to give a percent inhibition.

$$\frac{\text{Total combined length of Replicates}}{\text{Number of Replicates}} = \text{Average Length}$$

$$\text{Avg. Length} - 10\ \text{cm} = \text{Avg. Increased Growth}$$

$$\frac{\text{Avg. Increased Growth}}{\text{Avg. Increased Growth SC}} \times 100 = \%\ \text{Inhibition}$$

Several of the compounds of the invention were evaluated as aquatic herbicides by employing three aquatic weed species. The compounds were formulated for evaluation as described above, and the herbicidal activity was determined by visual observations based upon non-treated controls. Activity ratings were made on a scale of 1 to 5 as follows:

1 = no observable effect
2 = slight plant injury
3 = moderate plant injury
4 = 50-90% plant injury
5 = plants completely killed

A = abscission D = stem disintegration
C = chlorosis N = necrosis

The results of this test are reported in Table 2.

## Table 2

### Aquatic Herbicide Activity

| Compound Evaluated | Concentration ppm | 1-week post treatment Hydrilla | 1-week post treatment Coontail | 1-week post treatment Duckweed | 2-week post treatment Hydrilla | 2-week post treatment Coontail | 2-week post treatment Duckweed | 3-week post treatment Hydrilla | 3-week post treatment Coontail | 3-week post treatment Duckweed |
|---|---|---|---|---|---|---|---|---|---|---|
| 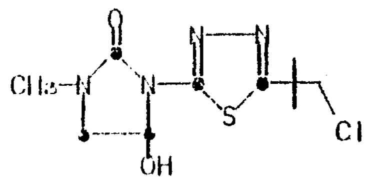 | 10 | 1 | 1 | 1 | 4D | 3C | 2C | 5D | 3C | 4C |
| | 4 | 1 | 1 | 1 | 1 | 1 | 4C | 2D | 2C | 5C |
| | 2 | 1 | 1 | 1 | 1 | 1 | 4C | 2D | 2C | 4C |
| 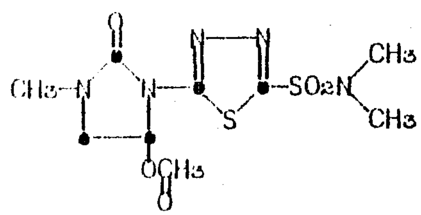 | 10 | 1 | 1 | 1 | 2N | 3C | 2C | 5D | 4C | 4C |
| | 4 | 1 | 1 | 1 | 5D | 5D | 3C | 5D | 5D | 4C |
| | 2 | 1 | 1 | 1 | 4D | 4A | 4C | 5D | 5D | 3C |
| 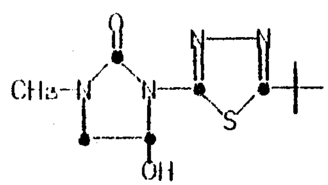 | 10 | 1 | 1 | 1 | 4D | 3C | 2C | 5D | 3C | 4C |
| | 4 | 1 | 1 | 1 | 1 | 1 | 3C | 3D | 2C | 5C |
| | 2 | 1 | 1 | 1 | 1 | 3A | 4C | 2C | 4C | 4C |

Table 2 continued

| Compound Evaluated | Concentration ppm | 1-week post treatment Hydrilla | Coontail | Duckweed | 2-week post treatment Hydrilla | Coontail | Duckweed | 3-week post treatment Hydrilla | Coontail | Duckweed |
|---|---|---|---|---|---|---|---|---|---|---|
| 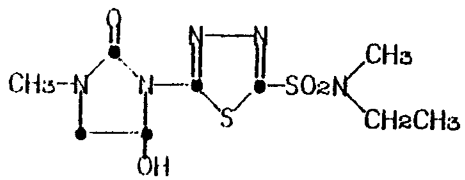 | 10 | 1 | 1 | 1 | 5D | 4C | 2C | 5D | 4D | 4C |
| | 4 | 1 | 1 | 1 | 4D | 3C | 3C | 5D | 3C | 4C |
| | 2 | 1 | 1 | 1 | 2C | 2C | 4C | 4D | 3C | 4C |
| 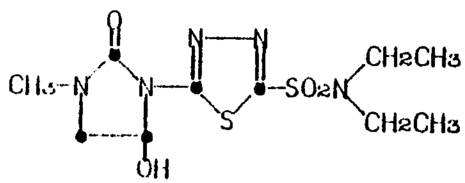 | 10 | 1 | 1 | 1 | 5D | 4A | 2C | 5D | 5D | 4C |
| | 4 | 1 | 1 | 1 | 1 | 1 | 3C | 2N | 2C | 5C |
| | 2 | 1 | 2C | 1 | 2C | 2C | 4C | 4D | 3C | 4C |
| 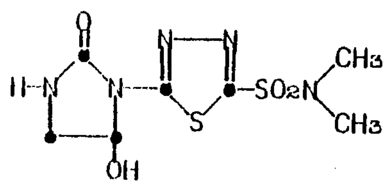 | 10 | 1 | 1 | 1 | 1 | 1 | 1 | 1 | 2C | 1 |
| | 4 | 1 | 1 | 1 | 1 | 1 | 1 | 2N | 1 | 1 |
| | 2 | 1 | 1 | 1 | 1 | 2C | 1 | 1 | 1 | 1 |
| Solvent Control | 10 | 1 | 1 | 1 | 1 | 1 | 1 | 1 | 1 | 2C |
| | 4 | 1 | 2C | 1 | 1 | 2C | 1 | 2N | 2C | 2C |
| | 2 | 1 | 1 | 1 | 1 | 2C | 1 | 1 | 3C | 1 |

The compounds of the invention are also expected to be useful in the control of water hyacinth. Water hyacinth is a floating aquatic plant of the order Eichhornia crassipes of the family Pontederiaceae. The plant is very troublesome in warm regions since it floats on water and often clogs waterways, and is very difficult to control safely.

As the data presented in the Tables above demonstrate, the compounds defined herein are useful in the control of aquatic vegetative growth. A preferred embodiment of this invention is a method of eliminating and controlling the growth of aquatic plants. This method is practiced by adding the active thiadiazolyl imidazolidinone derivatives to the water containing the submerged, emergent, ditchbank or floating aquatic plants, or otherwise contacting the plants with the active compounds, for example, by applying the compounds to the sub-aqueous soil in which the aquatic plants are rooted. Also provided by this invention are aquatic formulations comprising a thiadiazolylimidazolidinone derivative defined herein together with an aquatically acceptable carrier. Such aquatic formulations will contain from about 1 to about 90 percent by weight of the active imidazolidinone derivative. The compounds may be applied to the water when formulated as dusts by being admixed with a powdered solid carrier such as bentonite, Fuller's earth, diatomaceous earth, or various mineral silicates, e.g., mica, talc, pyrophyllite, and clays. The compounds may also be mixed with surface-active dispersing agents to form concentrates to facilitate dispersion in water and to improve

the wetting properties when used as sprays. If desired, the compounds may be mixed with a powdered solid carrier, together with a surface-active dispersing agent, so that a wettable powder may be obtained which may be applied directly, or which may be shaken with water to make an aqueous dispersion for application in that form. These wettable powder formulations suitably contain from about 25 to about 85 percent by weight of the active ingredient, i.e., an aquatic growth regulating compound coming within the scope of the generic formulae, supra. The compounds may be dissolved in an oil, such as a hydrocarbon or chlorinated hydrocarbon oil, and the oil solution of the compound dispersed in water with the aid of a surface-active dispersing agent to give a sprayable aqueous dispersion. Such surface-active dispersing agents may be anionic, nonionic, or cationic surface-active agents. Such surface-active agents are well-known, and reference is made to Hoffman et al., U.S. Patent No. 2,614,916, columns 2-4, for detailed examples of the same. The compounds useful in this embodiment of the invention may also be applied by the aerosol method. Solutions for the aerosol treatment may be prepared by dissolving the compound directly in the aerosol carrier, which is a liquid under pressure, but which is a gas at ordinary temperature (e.g. 20°C.) and atmospheric pressure; or, the aerosol solution may be prepared by first dissolving the compound in a less volatile solvent, and then admixing such solution with the highly volatile liquid aerosol carrier.

Further, the compounds useful as aquatic growth regulators and aquatic herbicides can also be

applied in an invert emulsion formulation. An invert emulsion formulation is prepared by first making a solution of an aquatic growth regulating compound in heavy oils, such as diesel fuel, inverting oil, and the like, and combining the thus-obtained solution with water under high shear stirring. The thick emulsion is placed in the water and sinks to the bottom of the lake, river, pond, or the like, and the aquatic growth regulator is gradually released to control the growth of the aquatic plants. The following is an example of an invert emulsion formulation, prepared using the compound of Example No. 2 of this application.

Example 17

Invert Emulsion

| | |
|---|---|
| Compound of Example No. 2 | 12.5 gm |
| Diesel fuel | 333 ml |
| Inverting oil* | 333 ml |

*Vioko-Rhap Inverting Oil (Rhodia, Inc.)

Two-hundred fifty milliliters of this solution is combined with 3750 ml. of water under high shear stirring to give a thick invert emulsion.

The compounds useful as aquatic growth regulators and aquatic herbicides can also be applied as pellets which are prepared from a mixture of about 5% of the active ingredient, about 85% clay, and about 10% water, all percentages being by weight. The mixture is then extruded through a pellet mill using a suitably sized die, e.g., about 1/8 in. diameter. The extruded pellets are about 1/8 in. by 1 1/2 in., and are then dried to about 8% moisture content.

Accordingly, one aspect of the invention is that it provides a formulation for regulating aquatic plant growth comprising as an active ingredient from 1 to 90 wt. % of a compound of formula (I):

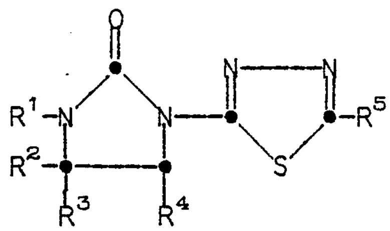

(I)

wherein:

$R^1$ is hydrogen, $C_1$-$C_6$ alkyl, $C_2$-$C_6$ alkenyl, $C_2$-$C_6$ alkynyl, or halo-$C_1$-$C_6$ alkyl;

$R^2$ is hydrogen, or taken together with $R^4$ completes a double bond;

$R^3$ is hydrogen, $C_1$-$C_6$ alkyl, hydroxy, $C_1$-$C_6$ alkoxy, or $C_1$-$C_6$ alkanoyloxy;

$R^4$ is hydroxy, $C_1$-$C_6$ alkoxy, $C_1$-$C_6$ alkanoyloxy, or taken together with $R^2$, $R^4$ completes a double bond;

$R^5$ is $C_1$-$C_6$ alkyl, $C_2$-$C_6$ alkenyl, $C_2$-$C_6$ alkynyl, halo-$C_1$-$C_6$ alkyl, $C_1$-$C_6$ alkylthio, $C_1$-$C_6$ alkylsulfinyl, $C_1$-$C_6$ alkylsulfonyl, or $SO_2NR^6R^7$ wherein

$R^6$ is hydrogen or $C_1$-$C_6$ alkyl;

$R^7$ is $C_1$-$C_6$ alkyl, $C_1$-$C_6$ alkoxy, $C_2$-$C_6$ alkenyl, or $C_2$-$C_6$ alkynyl, associated with at least one aquatically acceptable carrier or diluent therefor.

The method of controlling aquatic plant growth provided by this invention is practiced by

adding to the water containing the submerged or floating plants a growth-regulating or herbicidal amount of one of the herein-disclosed compounds, such that a concentration of from about 0.01 to about 10 ppm of the active compound is attained. A preferred method of aquatic plant growth regulation provided by this invention is directed toward the control of plants such as water hyacinth. Such plants can be controlled by foliar or root application of a compound of this invention at a rate of about 0.01 to about 1.0 pounds per acre (about 0.011 to about 1.1 kg/ha).

The optimum concentration of active compound for any specific aquatic weed control problem varies with the temperature, the species to be controlled, and the shape of the body of water to be treated. At higher water temperatures, less compound is generally required for a given degree of control than is needed at lower temperatures. When used to control algae or similar aquatic plant growth, the compounds will usually be employed at concentrations of about 0.1 to about 10 ppm. In terms of pounds of compound per acre of water one foot deep, 0.1 to 10 ppm is equal to about 0.3 to about 30 pounds per acre of water one foot deep.

The thiadiazolylimidazolidinones employed in the method of this invention can be used in combination with other aquatic growth regulators and aquatic herbicides. Typical agents to be employed in combination treatments include fluridone, diuron, cutrine-plus, aquazine, and the like. A preferred combination for use according to this invention is a thiadiazolylimidazolidinone and fluridone.

## CLAIMS

1. A method for controlling the growth of aquatic plants comprising contacting the plants or the water in which the plants are growing with an aquatic herbicidally-effective amount of a compound of the formula (I):

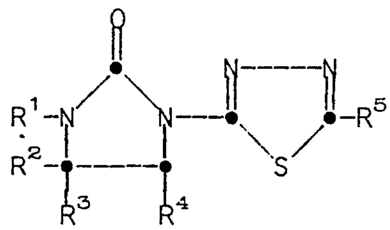

(I)

wherein:

$R^1$ is hydrogen, $C_1$-$C_6$ alkyl, $C_2$-$C_6$ alkenyl, $C_2$-$C_6$ alkynyl, or halo-$C_1$-$C_6$ alkyl;

$R^2$ is hydrogen, or taken together with $R^4$ completes a double bond;

$R^3$ is hydrogen, $C_1$-$C_6$ alkyl, hydroxy, $C_1$-$C_6$ alkoxy, or $C_1$-$C_6$ alkanoyloxy;

$R^4$ is hydroxy, $C_1$-$C_6$ alkoxy, $C_1$-$C_6$ alkanoyloxy, or taken together with $R^2$, $R^4$ completes a double bond;

$R^5$ is $C_1$-$C_6$ alkyl, $C_2$-$C_6$ alkenyl, $C_2$-$C_6$ alkynyl, halo-$C_1$-$C_6$ alkyl, $C_1$-$C_6$ alkylthio, $C_1$-$C_6$ alkylsulfinyl, $C_1$-$C_6$ alkylsulfonyl, or $SO_2NR^6R^7$ wherein

$R^6$ is hydrogen or $C_1$-$C_6$ alkyl;

$R^7$ is $C_1$-$C_6$ alkyl, $C_1$-$C_6$ alkoxy, $C_2$-$C_6$ alkenyl, or $C_2$-$C_6$ alkynyl.

2. The method of claim 1 employing a compound of formula (I) wherein $R^1$ is $C_1$-$C_6$ alkyl, $R^2$ and $R^3$ are both hydrogen, $R^4$ is hydroxy and $R^5$ is $C_1$-$C_6$ alkyl or $SO_2NR^6R^7$ where $R^6$ and $R^7$ independently are $C_1$-$C_6$ alkyl.

3. A formulation for regulating aquatic plant growth comprising as an active ingredient from 1 to 90 wt. % of a compound of formula (I):

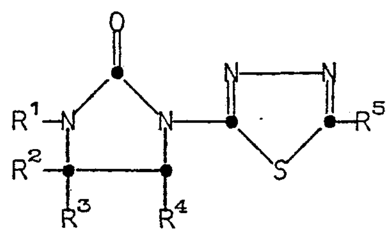

(I)

wherein:

$R^1$ is hydrogen, $C_1$-$C_6$ alkyl, $C_2$-$C_6$ alkenyl, $C_2$-$C_6$ alkynyl, or halo-$C_1$-$C_6$ alkyl;

$R^2$ is hydrogen, or taken together with $R^4$ completes a double bond;

$R^3$ is hydrogen, $C_1$-$C_6$ alkyl, hydroxy, $C_1$-$C_6$ alkoxy, or $C_1$-$C_6$ alkanoyloxy;

$R^4$ is hydroxy, $C_1$-$C_6$ alkoxy, $C_1$-$C_6$ alkanoyloxy, or taken together with $R^2$, $R^4$ completes a double bond;

$R^5$ is $C_1$-$C_6$ alkyl, $C_2$-$C_6$ alkenyl, $C_2$-$C_6$ alkynyl, halo-$C_1$-$C_6$ alkyl, $C_1$-$C_6$ alkylthio, $C_1$-$C_6$ alkylsulfinyl, $C_1$-$C_6$ alkylsulfonyl, or $SO_2NR^6R^7$ wherein

$R^6$ is hydrogen or $C_1$-$C_6$ alkyl;

$R^7$ is $C_1$-$C_6$ alkyl, $C_1$-$C_6$ alkoxy, $C_2$-$C_6$ alkenyl, or $C_2$-$C_6$ alkynyl associated with at least one aquatically acceptable carrier or diluent therefor.

4. The formulation of claim 3 including an active ingredient of formula (I) wherein $R^1$ is $C_1$-$C_6$ alkyl, $R^2$ and $R^3$ are hydrogen, $R^4$ is hydroxy and $R^5$ is $C_1$-$C_6$ alkyl or $SO_2NR^6R^7$ where $R^6$ and $R^7$ independently are $C_1$-$C_6$ alkyl.

5. The formulation of claim 4 wherein $R^5$ is $SO_2NR^6R^7$.

6. The formulation of claim 3 or 4 employing a compound of formula (I) wherein $R^5$ is 1,1-dimethylethyl or tert-butyl.

7. The formulation of any one of claims 3-6 employing a compound of formula (I) wherein $R^7$ is ethyl, n-propyl, or n-butyl.

8. The formulation of any one of claims 3-7 employing a compound of formula (I) wherein $R^1$ is methyl.

9. A formulation of any one of claims 3, and 6-8 employing a compound of formula (I) wherein $R^4$ is acetoxy.

0094181

European Patent Office

# EUROPEAN SEARCH REPORT

Application number

EP 83 30 2430

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | CHEMICAL ABSTRACTS, vol. 89, no. 15, 9th October 1978, page 172, no. 124469s, Columbus, Ohio, USA N. DECHORETZ et al.: "Evaluation of velpar, buthidazole and fluridone for the control of aquatic weeds" & PROC. WEST. SOC. WEED SCI. 1978, 31, 111-116 * Abstract * | 1-4,6, 8 | A 01 N 43/82 // C 07 D 41/704 C 07 D 285/12 |
| | ----- | | TECHNICAL FIELDS SEARCHED (Int. Cl. 3) |
| | | | A 01 N 43/00 C 07 D 417/00 C 07 D 285/00 |

The present search report has been drawn up for all claims

| Place of search | Date of completion of the search | Examiner |
|---|---|---|
| THE HAGUE | 16-08-1983 | CREMERS K. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document